# EUROPEAN PATENT SPECIFICATION

(11) **EP 1 487 508 B1**
(45) Date of publication and mention of the grant of the patent: **10.05.2006**
(21) Application number: 03708359.9
(22) Date of filing: 24.03.2003
(51) Int. Cl.: A61L 15/28, A61L 15/42

(54) **THERAPEUTIC COMPOSITIONS COMPRISING MODIFIED POLYSACCHARIDES**
THERAPEUTISCHE ZUBEREITUNGEN ENTHALTEND MODIFIZIERTE POLYSACCHARIDE
COMPOSITIONS THERAPEUTIQUES COMPRENANT DES POLYSACCHARIDES MODIFIES

(30) Priority: 25.03.2002 GB 0207008
(43) Date of publication of application: 22.12.2004
(73) Proprietor: Johnson & Johnson Medical Ltd., Edinburgh EH2 4NH (GB)
(72) Inventor: SCHMIDT, Ryszard, Jan, Barnoldswick BB18 6HJ (GB)
(74) Representative: James, Anthony Christopher W.P.
(86) International application number: PCT/GB2003/001208
(87) International publication number: WO 2003/080135

(56) References cited:
- EP-A- 0 556 110
- WO-A-94/04129
- WO-A-94/13333
- CONSTANTIA E. K. ET AL: "Thiolated polymers - thiomers: development and in vitro evaluation of chitosan-thioglycolic acid conjugates" BIOMATERIALS, vol. 22, 2001, pages 2345-2352, XP002242794
- BERNKOP-SCHNÜRCH A. ET AL: "Synthesis and characterisation of mucoadhesive thiolated polymers" INTERNATIONAL JOURNAL OF PHARMACEUTICS, vol. 194, 2000, pages 239-247, XP002242795
- CONSTANTIA E. K. ET AL: "Polymer - cysteamine conjugates: new mucoadhesive excipients for drug delivery" INTERNATIONAL JOURNAL OF PHARMACEUTICS, vol. 234, 2 March 2002 (2002-03-02), pages 91-99, XP002242796

## Description

The present invention relates to the use of chemically modified polysaccharides in the preparation of dressings for therapeutic applications such as wound treatment.

Concentrations of reactive oxygen species such as hydroxyl radicals (-OH), singlet oxygen (¹O₂), hydroperoxyl radicals (·OOH), superoxide radical anions (·O₂⁻), and hydrogen peroxide (H₂O₂) can rise in damaged tissues, producing a condition known as oxidative stress. The presence of a low level of reactive oxygen species may be advantageous in the early stages of wound healing by both attracting and activating macrophages which engulf and kill bacteria and release cytokines and growth factors. However, prolonged and more severe oxidative stress may delay healing because it will produce chronic inflammation, divert available energy supply towards antioxidant defence at the expense of tissue reconstruction, and increase levels of matrix metalloproteinases which cause tissue breakdown. In more severe cases, elevated levels of reactive oxygen species can give rise to hydrogen peroxide-induced senescence or apoptosis (that is, programmed cell death) or tissue necrosis (that is, uncontrolled cell death and therefore permanent tissue damage).

Under mild oxidative stress, it is thought that hydrogen peroxide (H₂O₂) is the dominant species present, being formed rapidly from superoxide by the enzyme superoxide dismutase. This enzyme-mediated dismutation reaction also minimises the production of singlet oxygen that can arise when superoxide is produced too rapidly and therefore has the opportunity to dismutate spontaneously without enzyme assistance. Rapid enzyme-mediated dismutation of superoxide also minimises levels of hydroperoxyl radical, the non-ionised form of superoxide. Levels of hydrogen peroxide are in turn kept low by the actions of catalase and glutathione peroxidase. Thus, under mild oxidative stress conditions when hydrogen peroxide levels are slightly raised (around 10⁻⁸ to 10⁻⁴ molar), it has been found that the rate of cell proliferation in fibroblast cultures is stimulated.

Accordingly, the healing of chronic wounds may be assisted by the use of antioxidant wound dressings that react specifically with excess reactive oxygen species such as those listed above and hence reduce the level of oxidative stress.

US-A-5667501 describes compositions comprising chemically modified polymers grafted with chemical groups that confer antioxidant activity as measured by a diphenylpicrylhydrazyl (DPPH) test and that also generate low levels of hydrogen peroxide by reaction with molecular oxygen in the wound bed to stimulate macrophage activity and fibroblast proliferation. The compositions may be used to promote the healing of chronic wounds. Preferably, the polymer is a polymer bearing hydroxyl, carbonyl or amide functional groups, or a polysaccharide bearing hydroxyl functional groups, said functional groups having been converted to derivatives that are persistent free radicals or precursors of persistent free radicals, that is to say they are free radical scavenging antioxidant groups.

US-A-5612321 describes compositions comprising polysaccharides grafted with antioxidants on at least one hydroxyl group of the polysaccharide. The compositions may be used *inter alia* to promote the healing of chronic wounds. Preferably, the polysaccharide is hyaluronic acid and the antioxidant group comprises a phenol group.

Constantia E. Kast *et al*. in Biomaterials, vol. 22 (2001), pages 2345-2352 and C. E. Kast *et al*. in International Journal of Pharmaceutics, vol. 234 (2002), pages 91-99 describe thiol-modified chitosan for use as a mucoadhesive drug delivery system. There is no disclosure that the thiol-modified chitosan has any antioxidant properties or that is is suitable for use in wound treatment.

A. Bemkop-Schnürch *et al*. in International Journal of Pharmaceutics, vol. 194 (2000), pages 239-247 describe studies on thiol-modified polysaccharides for use as mucoadhesive drug delivery systems. There is no disclosure that the thiol-modified polysaccharides used had antioxidant properties, nor is there any disclosure of the use of such modified polysaccharides in wound treatment.

In the complex biochemistry of infected and/or chronic wounds, it is also thought that iron plays an important role. For example, iron may play a role in catalysing the generation of the above-described reactive oxygen species, and thereby may contribute to wound chronicity. Furthermore, bacteria require iron for metabolism and can secrete siderophores for the purpose of scavenging iron. It therefore appears that removal of free iron from the wound fluid, preferably without removal of other dissolved species such as zinc that promote wound healing, could assist in reducing both wound colonisation or infection by bacteria and wound chronicity.

US-A-6156334 describes novel wound coverings comprising chelators such as the siderophore deferoxamine grafted onto a suitable carrier through a linker molecule, which remove interfering factors such as iron from the wound bed. Foitzik A *et al*. in the Proceedings of the Joint Meeting of the European Tissue Repair Society/The Wound Healing Society. Bordeaux, 24^{th} - 28^{th} August 1999, describe treatment of chronic venous ulcers by means of a gauze dressing having deferoxamine coupled thereto. The resulting iron-absorbing dressing is expensive to manufacture, and its antioxidant properties and affinity for beneficial species such as zinc are unknown.

EP-A-0556110 describes the use of thiol-grafted polymers to improve the adhesion of cosmetic and therapeutic compositions for application to the skin.

The present invention relates to the use of a chemically modified polysaccharide as defined in claim 1 for the preparation of a dressing for the treatment of wounds.

It has been found that the presence of thiol groups confers a surprising high affinity for iron with a low affinity for zinc and additionally a reactivity with free radicals that could render the derivatised polysaccharide useful for the removal of reactive oxygen species from the wound environment.

The thiol groups may replace hydroxyl groups of the saccharide units of the polysaccharide, or preferably the thiol groups are present on a side chain that is grafted onto the polysaccharide through an ester or ether linkage. Preferably, the side chain has from 1 to 8 carbon atoms. A preferred side chain is 2-ethoxythiol, for example in a modified polysaccharide comprising a moiety of the following general formula. 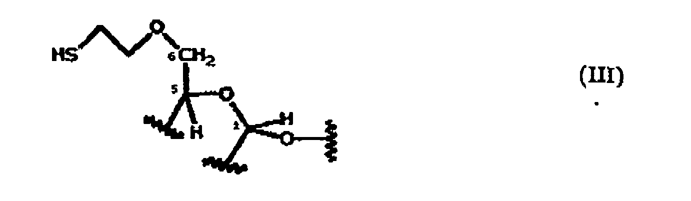 Such side chains of structure (III) can conveniently be formed by reacting a polysaccharide or a modified polysaccharide, for example hydroxyethyl cellulose, with Lawesson's reagent as described further below.

Preferably, the chemically modified polysaccharide according to the present invention is a modified or derivatised glycan or aminoglycan.

Preferably, the modified or derivatised glycan is a modified or derivatised guluronomannuronan such as an alginate or alginate derivative.

Preferably, the modified or derivatised glycan is a modified or derivatised glucan such as cellulose or a cellulose derivative.

More preferably, the modified or derivatised glycan is a hydroxyalkyl cellulose for example hydroxyethyl cellulose, and the modified or derivatised aminoglycan is a modified or derivatised chitin/chitosan.

Optionally, the modified polysaccharide may comprise a mixture of two or more modified glycans, aminoglycans or derivatives thereof.

At least 1% of the saccharide residues in the modified polysaccharide are modified in accordance with the invention by the introduction of a thiol group or a thiol-containing group. Preferably, at least 5% of the saccharide residues are so modified, and more preferably at least 10% to 20% of the saccharide residues are so modified.

Preferably, the modified polysaccharide used in the present invention has a free radical activity, that is to say an antioxidant activity, of at least 15% in the diphenylpicrylhydrazyl (DPPH) test, measured as percentage reduction in absorbance at 524nm after 4 hours of a 0.5%w/v dispersion of the polysaccharide in 10⁻⁴M DPPH, as described further hereinbelow in procedure 2. Preferably the percentage reduction in absorbance in the DPPH test is at least 25%, more preferably at least 50%, and most preferably at least 75%.

Preferably, the modified polysaccharide will absorb water or wound fluid and hence become wet, swell or become a gelatinous mass but will not spontaneously dissolve or disperse therein. That is to say, it is hydrophilic but has a solubility of preferably less than 1g/liter in water at 25°C. Low solubility renders such polysaccharides especially suitable for use as wound dressings to remove iron and reactive oxygen species from the wound fluid.

The modified polysaccharide used in the present invention preferably forms complexes with iron selectively over zinc. That is to say, the molar fraction of the iron ions absorbed by the modified polysaccharide in the method of Procedure 1 is higher than the fraction of the zinc ions absorbed. More preferably, the stability constant of the polysaccharide complex with Fe³⁺ ions is at least ten times the stability constant of the polysaccharide complex with Zn²⁺ ions. Preferably, a complex of the polysaccharide with Fe³⁺ in water at neutral Ph has a stability constant of at least 10³, preferably at least 10⁶.

The wound dressing and/or the modified polysaccharide used in the present invention may be provided in the form of beads, flakes, powder, and preferably in the form of a film, a fibrous pad, a web, a woven or non-woven fabric, a freeze-dried sponge, a foam or combinations thereof. Such formats are especially useful for wound dressings, which can easily be made by derivatisation of or by grafting of side chains onto polysaccharides already provided in the relevant formats.

The antioxidant and iron sequestering properties of the polysaccharides used in the present invention suggest applications in a range of medical applications, including the treatment of acute surgical and traumatic wounds, burns, fistulas, venous ulcers, arterial ulcers, pressure sores (otherwise known as decubitus ulcers), diabetic ulcers, ulcers of mixed aetiology, and other chronic or necrotic wounds and inflammatory lesions and disorders. Another therapeutic application that is likely to be useful is in the treatment or prevention of lipodermatosclerosis (LDS). LDS is a pre-ulcerous discoloring and hardening of the skin in patients suffering from venous insufficiency leading ultimately to varicose ulcers. It is thought that excess iron plays a part in the mechanism of LDS, and therefore that the iron sequestering properties of these modified polysaccharides will be useful in the treatment and prevention of LDS.

In addition to the solid formats discussed above, the wound dressing composition may also be in the form of a gel or viscous fluid for topical application.

Preferably, the wound dressing composition comprises at least about 1wt.% of the thiolated polysaccharide, more preferably at least about 10wt.%, more preferably at least about 50wt% of the thiolated polysaccharide. In certain embodiments, the wound dressing consists essentially of the thiolated polysaccharide.

Preferably, the wound dressing is sterile, and preferably it is packaged in a microorganism impermeable container.

Preferably, the wound is a chronic wound. More preferably, the chronic wound is selected from the group consisting of ulcers of venous, arterial or mixed aetiology, decubitus ulcers, or diabetic ulcers.

The present invention further provides the use of a chemically modified polysaccharide, wherein the polysaccharide has been chemically modified by converting one or more hydroxyl groups on the saccharide units of the modified polysaccharide into thiol groups, or by providing thiol groups on side chains grafted onto the saccharide units of the modified polysaccharide, and wherein at least 1% of the saccharide residues of the modified polysaccharide comprise a thiol group or a side chain bearing a thiol group, for the preparation of a medicament for the treatment or prevention of lipodermatosclerosis (LDS).

The treatment or prevention of lipodermatosclerosis in a mammal comprises applying to LDS skin, or to skin liable to develop LDS, a therapeutically effective amount of the medicament. Skin liable to develop LDS would include, for example, the lower leg of individuals suffering from venous insufficiency or varicose veins.

The modified polysaccharides used in the present invention may be provided by a processcomprising the step of reacting a polysaccharide or polysaccharide derivative with Lawesson's reagent having the structure (V):

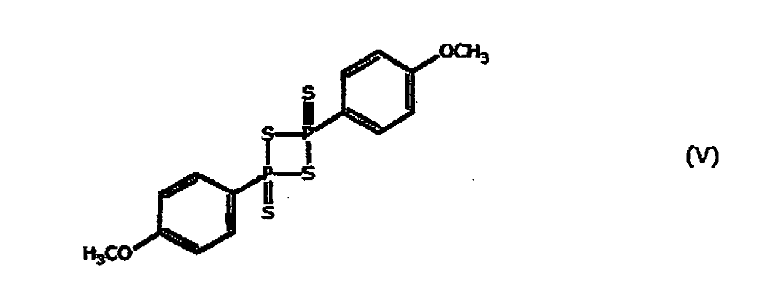

Preferably, the Lawesson's reagent is reacted with said polysaccharide or polysaccharide derivative in an amount of at least 0.1 moles per mole of saccharide residues of the polysaccharide or polysaccharide derivative.

Specific embodiments of the present invention will now be described further with reference to the drawings, in which:

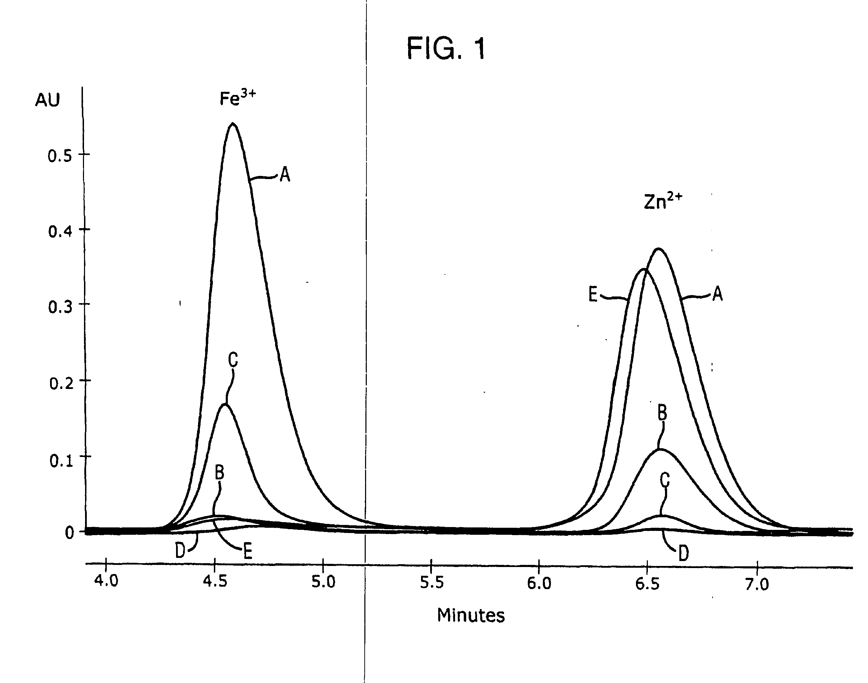

Figure 1 shows chromatograms of iron and zinc ion concentrations for a control serum (D) containing no added iron or zinc, a control serum containing added iron and zinc ions (A), and the serum A after treatment with a thiolated polysaccharide according to the present invention (E). The plots B and C relate to reference examples that are not considered further here.

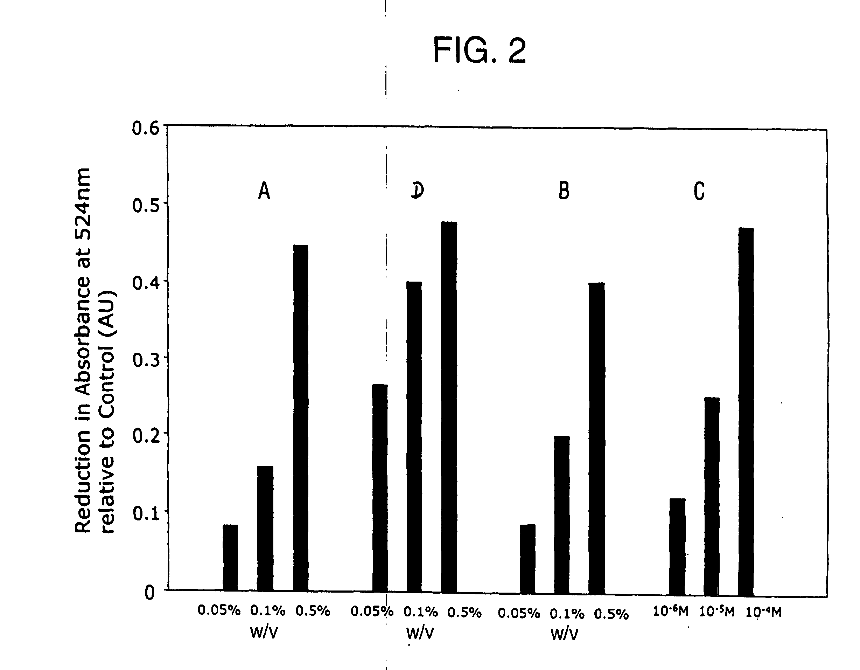

Figure 2 shows the absolute reduction in absorbance of DPPH at 524nm versus concentration of antioxidant for ascorbic acid (C, reference example), and a thiolated polysaccharide according to the invention (D). The absorbance of the starting DPPH solution was 0.506AU. Plots A and B relate to reference examples not considered further here.

### Reference Example 1

A sample of hydroxyethyl cellulose was modified by treatment with Lawesson's reagent as follows. Hydroxyethyl cellulose (10.3g, 50mmoles) was placed in a dry 250ml three-neck round bottomed flask with 150ml of anhydrous toluene. To this was added Lawesson's reagent (Aldrich Chemical Co. catalog number 22,743-9; 2.02g; 5mmoles). The flask was fitted with a water condenser and the mixture was then stirred and heated under reflux in a nitrogen atmosphere for 4 hours. The reaction mixture was then allowed to cool to 30°C before quenching with aqueous 1M potassium hydroxide (KOH) solution (100ml) to ensure complete hydrolysis of Lawesson's reagent After standing for a further 15 minutes, liquid phases were decanted and collected for disposal. The remaining solid material was washed with aqueous 1 M KOH (100ml x 3) and with aqueous 1M hydrochloric acid (100ml x 3), decanting the washings between each aliquot of wash solution. The remaining solid material was then washed with deionised water (100ml x 3) and with ethanol (100ml x 3)- Centrifugation was necessary between washings. The resultant pellets were placed on a tray and blast frozen before freeze drying. Yield of product was 4.03g. The degree of thiolation could be increased by increasing the initial proportion of Lawesson's reagent to polysaccharide; loss of product during washing could be decreased by using isopropanol/water mixtures as solvents for the alkaline, acidic, and neutral washes.

The thiolated hydroxyethyl cellulose exhibited a high affinity for Fe³⁺ and a particularly low affinity for Zn²⁺ and also antioxidant properties as determined in procedures 1 and 2 described below. The results for this material are shown as graph E in Fig. 1 and as plot D in Fig. 2. Neither of these properties is observed for the unmodified hydroxyethyl cellulose starting material.

### Procedure 1

The ability of modified polysaccharides according to the present invention to sequester iron ions selectively over zinc ions in aqueous media was determined as follows.

The modified polysaccharide was added with stirring to a solution containing iron and/or zinc ions in a suitable matrix such as buffer, serum-containing buffer or cell culture medium that is intended to simulate wound fluid. Following a suitable incubation period, ion chromatography was used to determine the levels of uncomplexed Fe³⁺ and/or Zn²⁺ ions remaining in solution.

In the present example, solutions of FeCl₃.6H₂O and ZnSO₄.7H₂O in water were added to Dulbecco's Modified Eagle's Medium (DMEM; Sigma Chemical Co. catalog number D 5546) containing 10% v/v calf serum (Sigma Chemical Co. catalog number N 4637) to produce final concentrations of 50 ppm Fe³⁺ and 50 ppm Zn²⁺. The mixed solution was incubated at 37°C with gentle agitation for 16 hours with the modified polysaccharide present at a concentration of 2% w/v. The samples were then centrifuged, and a 0.9ml sample of the supernatant was analysed for iron and zinc content by the following method.

The centrifuged solution was treated with 0.1ml of a 20% w/v solution of trichloroacetic acid (TCA) to give a final concentration of 2% w/v TCA. The tube was vortexed for 10 seconds and then centrifuged for 15 minutes or longer to remove solids. The supernatant (0.5ml) was added to a clean dry HPLC vial to which was added 0.5ml of 0.5M nitric acid (HNO₃) prepared in deionised water.

The samples were then analysed for free iron and zinc ions using a Dionex DX500 HPLC apparatus according to the following method details:
- Instrument:: Dionex DX500 HPLC
- Column:: Ionpac CS5A Analytical Column (Dionex part no. 046100)
lonpac CS5A Guard Column (Dionex part no. 046104)
- Eluants:: A - Deionised water
B - Metpac PDCA reagent concentrate (part no. 046088)
C - Metpac PAR reagent diluent (Dionex part no. 046094)
- Postcolumn Reagent:: 4-(2-pyridylazo) resorcinol monosodium salt (PAR) 0.12g/l Detector
- Wavelength:: 530nm
- Temperature:: 25°C
- Flow rate:: 1.2ml.min (80% eluant A: 20% eluant B) in column; 0.6ml/min eluant C postcolumn
- Sample size:: 100 µl

Data for the samples prepared in accordance with Examples 1 are shown in Figure 1. It can be seen that the modified polysaccharide removes Fe³⁺ and Zn²⁺ ions from Dulbecco's Modified Eagle's Medium containing calf serum with varying selectivities.

### Procedure 2

The ability of the modified polysaccharide materials to react with and remove oxygen containing free radicals is assessed by the DPPH test described in WO94/13333, the entire content of which is expressly incorporated herein by reference. The test is adapted from that described by Blois M.S. in Nature 181: 1199 (1958), and Banda P.W. *et al*., in Analytical Letters 7: 41 (1974).

Briefly, modified polysaccharide under test (2.5mg; 5mg; & 25mg sample sizes) was suspended in 2.5ml of 0.1 M pH 7.0 phosphate buffer. A solution of diphenylpicrylhydrazyl (DPPH) in methanol (10⁻⁴ M) was added in an amount of 2.5 ml and the mixture was shaken and stored in the dark at 20°C. The samples were assessed by measurement of their light absorbance at 524nm over 6 hours in comparison with a control, particular attention being paid to the figure after 4 hours. The percentage reduction of absorbance relative to the control after 4 hours gives the DPPH test value, with a reproducibility generally of ±5%. This value may conveniently be expressed in terms of a simple reduction in absorbance units (AU) relative to the control as shown in Figure 2 in which the DPPH control solution containing no test sample produced an absorbance reading of 0.506 AU. Ascorbic acid, a well known antioxidant, provides a useful positive control substance for comparative purposes.

Application of this test to the products of Examples 1-3 resulted in DPPH test values of 80 - 90% for the positive control (10⁻⁴M) and for the material of Example 3 at its highest concentration (0.5% w/v) after 4 hours, as shown in Fig. 2. In contrast, the unmodified hydroxyethyl cellulose exhibited much lower DPPH values of less than 15%.

The above embodiments have been described by way of example only. Many other embodiments falling within the scope of the accompanying claims will be apparent to the skilled reader.

## Claims

1. Use of a chemically modified polysaccharide, wherein the polysaccharide has been chemically modified by converting one or more hydroxyl groups on the saccharide units of the modified polysaccharide into thiol groups, or by providing thiol groups on side chains grafted onto the saccharide units of the modified polysaccharide, and wherein at least 1 % of the saccharide residues of the modified polysaccharide comprise a thiol group or a side chain bearing a thiol group, for the preparation of a dressing for the treatment of wound.

2. Use according to claim 1, wherein the modified polysaccharide is modified chitin/chitosan or a modified cellulose or a modified hydroxyalkyl cellulose or a modified alginate.

3. Use according to claim 1, wherein at least 5% of the saccharide residues of the modified polysaccharide comprise a thiol group or a side chain bearing a thiol group.

4. Use according to any preceding claim, wherein the modified polysaccharide has a free radical activity in the diphenylpicrylhydrazyl (DPPH) test for antioxidant activity as herein defined of at least 15%.

5. Use according to any preceding claim, wherein the modified polysaccharide is substantially insoluble in water.

6. Use according to any preceding claim, wherein the modified polysaccharide complexes with Fe³⁺ ions selectively over Zn²⁺ ions.

7. Use according to any preceding claim wherein the wound dressing is in the form of beads, flakes, powder, a film, a fibrous pad, a web, a woven or non-woven fabric, a freeze-dried sponge, a foam or any combination thereof.

8. Use according to any preceding claim, wherein the wound is a chronic wound.

9. Use according to any preceding claim, wherein the chronic wound is selected from the group consisting of ulcers of venous, arterial or mixed aetiology, decubitus ulcers, or diabetic ulcers.

10. Use of a chemically modified polysaccharide, wherein the polysaccharide has been chemically modified by converting one or more hydroxyl groups on the saccharide units of the modified polysaccharide into thiol groups, or by providing thiol groups on side chains grafted onto the saccharide units of the modified polysaccharide, and wherein at least 1% of the saccharide residues of the modified polysaccharide comprise a thiol group or a side chain bearing a thiol group, for the preparation of a medicament for the treatment or prevention of lipodermatosclerosis.

## Patentansprüche

1. Verwendung eines chemisch modifizierten Polysaccharids, wobei das Polysaccharid durch Umwandlung einer oder mehrerer Hydroxylgruppen auf den Saccharid-Einheiten des modifizierten Polysaccharids in Thiolgruppen oder durch Bereitstellung von Thiolgruppen auf Seitenketten, die auf die Saccharid-Einheiten des modifizierten Polysaccharids aufgepfropft sind, chemisch modifiziert worden ist und wobei wenigstens 1% der Saccharid-Reste des modifizierten Polysaccharids eine Thiolgruppe oder eine Seitengruppe, die eine Thiolgruppe trägt, umfassen, zur Herstellung eines Verbandes für die Behandlung von Wunden.

2. Verwendung nach Anspruch 1, **dadurch gekennzeichnet, daß** das modifizierte Polysaccharid modifiziertes Chitin/Chitosan oder eine modifizierte Cellulose oder eine modifizierte Hydroxyalkylcellulose oder ein modifiziertes Alginat ist.

3. Verwendung nach Anspruch 1, **dadurch gekennzeichnet, daß** wenigstens 5% der Saccharid-Reste des modifizierten Polysaccharids eine Thiolgruppe oder eine Seitenkette, die eine Thiolgruppe trägt, umfassen.

4. Verwendung nach einem vorangehenden Anspruch, **dadurch gekennzeichnet, daß** das modifizierte Polysaccharid eine Radikalaktivität im Diphenylpicrylhydrazyl(DPPH)-Test für Antioxidationsaktivität, wie hierin definiert, von wenigstens 15% besitzt.

5. Verwendung nach einem vorangehenden Anspruch, **dadurch gekennzeichnet, daß** das modifizierte Polysaccharid im wesentlichen unlöslich in Wasser ist.

6. Verwendung nach einem vorangehenden Anspruch, **dadurch gekennzeichnet, daß** das modifizierte Polysaccharid mit Fe³⁺-Ionen selektiv gegenüber Zn²⁺-Ionen komplexiert.

7. Verwendung nach einem vorangehenden Anspruch, **dadurch gekennzeichnet, daß** der Wundverband in der Form von Perlen, Schuppen, Pulver, einem Film, einem Faserkissen, einer Stoffbahn, einem gewebten oder nicht-gewebten Stoff, einem gefriergetrockneten Schwamm, einem Schaum oder einer Kombination derselben vorliegt.

8. Verwendung nach einem der vorangehenden Anspruch, **dadurch gekennzeichnet, daß** die Wunde eine chronische Wunde ist.

9. Verwendung nach einem vorangehenden Anspruch, **dadurch gekennzeichnet, daß** die chronische Wunde ausgewählt ist aus der Gruppe, die aus Ulcera venöser, arterieller oder gemischter Aetiologie, Dekubitalulcera oder Diabetesulcera besteht.

10. Verwendung eines chemisch modifizierten Polysaccharids, wobei das Polysaccharid durch Umwandlung einer oder mehrerer Hydroxylgruppen auf den Saccharid-Einheiten des modifizierten Polysaccharids in Thiolgruppen oder durch Bereitstellung von Thiolgruppen auf Seitenketten, die auf die Saccharid-Einheiten des modifizierten Polysaccharids aufgepfropft sind, chemisch modifiziert worden ist und wobei wenigstens 1% der Saccharid-Reste des modifizierten Polysaccharids eine Thiolgruppe oder eine Seitengruppe, die eine Thiolgruppe trägt, umfassen, zur Herstellung eines Arzneimittels zur Behandlung oder Prävention von Lipodermatosklerose.

## Revendications

1. Utilisation d'un polysaccharide modifié chimiquement, dans laquelle le polysaccharide a été modifié chimiquement en convertissant un ou plusieurs groupes hydroxyle sur les unités de saccharide du polysaccharide modifié en groupes thiol ou en proposant des groupes thiol sur des chaînes latérales greffées sur les unités de saccharide du polysaccharide modifié, et dans laquelle au moins 1 % des résidus de saccharide du polysaccharide modifié comprennent un groupe thiol ou une chaîne latérale portant un groupe thiol, pour la préparation d'un pansement pour le traitement d'une plaie.

2. Utilisation selon la revendication 1, dans laquelle le polysaccharide modifié est de la chitine modifiée/du chitosane modifié ou de la cellulose modifiée ou une hydroxyalkyl cellulose modifiée ou un alginate modifié.

3. Utilisation selon la revendication 1, dans laquelle au moins 5 % des résidus de saccharide du polysaccharide modifié comprennent un groupe thiol ou une chaîne latérale portant un groupe thiol.

4. Utilisation selon l'une quelconque des revendications précédentes, dans laquelle le polysaccharide modifié possède une activité anti-radicalaire dans le test au diphénylpicrylhydrazyle (DPPH) visant l'activité antioxydante telle que définie ici d'au moins 15 %.

5. Utilisation selon l'une quelconque des revendications précédentes, dans laquelle le polysaccharide modifié est substantiellement insoluble dans l'eau.

6. Utilisation selon l'une quelconque des revendications précédentes, dans laquelle le polysaccharide modifié forme un complexe avec les ions Fe³⁺ de manière sélective par rapport aux ions Zn²⁺.

7. Utilisation selon l'une quelconque des revendications précédentes, dans laquelle le pansement se présente sous la forme de billes, de plaques, de poudre, d'un film, d'une compresse fibreuse, d'une toile, d'un tissu tissé ou non tissé, d'une éponge lyophilisée, d'une mousse ou d'une quelconque combinaison de ceux-ci.

8. Utilisation selon l'une quelconque des revendications précédentes, dans laquelle la plaie est une plaie chronique.

9. Utilisation selon l'une quelconque des revendications précédentes, dans laquelle la plaie chronique est sélectionnée dans le groupe constitué des ulcères d'étiologie veineuse, artérielle ou mixte, des ulcères décubitus ou des ulcères diabétiques.

10. Utilisation d'un polysaccharide modifié chimiquement, dans laquelle le polysaccharide a été modifié chimiquement en convertissant un ou plusieurs groupes hydroxyle sur les unités de saccharide du polysaccharide modifié en groupes thiol ou en proposant des groupes thiol sur des chaînes latérales greffées sur les unités de saccharide du polysaccharide modifié, et dans laquelle au moins 1 % des résidus de saccharide du polysaccharide modifié comprennent un groupe thiol ou une chaîne latérale portant un groupe thiol, pour la préparation d'un médicament pour le traitement ou la prévention de la lipodermatosclérose.
